(19) Europäisches Patentamt
European Patent Office
Office européen des brevets

(11) Veröffentlichungsnummer: **0 129 506 B1**

# (12) EUROPÄISCHE PATENTSCHRIFT

(45) Veröffentlichungstag der Patentschrift: **06.03.91**

(51) Int. Cl.$^5$: **C07D 277/74**, C07D 263/58, C07D 235/28, C23F 11/16

(21) Anmeldenummer: **84810217.4**

(22) Anmeldetag: **08.05.84**

Die Akte enthält technische Angaben, die nach dem Eingang der Anmeldung eingereicht wurden und die nicht in dieser Patentschrift enthalten sind.

## (54) Heterocyclisch substituierte Thio(cyclo)alkanpolycarbonsäuren.

(30) Priorität: **14.05.83 GB 8313322**

(43) Veröffentlichungstag der Anmeldung:
**27.12.84 Patentblatt 84/52**

(45) Bekanntmachung des Hinweises auf die Patenterteilung:
**06.03.91 Patentblatt 91/10**

(84) Benannte Vertragsstaaten:
**BE CH DE FR GB IT LI NL SE**

(56) Entgegenhaltungen:
**EP-A- 0 003 817**
**EP-A- 0 093 908**
**CH-A- 458 048**
**DE-A- 3 341 633**
**GB-A- 1 028 924**

**CHEMICAL ABSTRACTS, Band 83, Nr. 25, 22. December 1975, Seite 396, Zusammenfassung Nr. 206268r, Columbus, Ohio, US; & JP-A-75 52 065**

(73) Patentinhaber: **CIBA-GEIGY AG**
**Klybeckstrasse 141**
**CH-4002 Basel(CH)**

(72) Erfinder: **Bentley, Robert Leonard, Dr.**
**34 Eddisbury Avenue**
**Urmston Manchester M31 2QJ(GB)**
Erfinder: **Savage, Michael Philip, Dr.**
**9 St. Ann's Square**
**Heald Green Cheadle Cheshire SK8 3EA(GB)**
Erfinder: **Shelton, Kenneth William, Dr.**
**52, Cranleigh Drive**
**Brooklands Sale Cheshire M33 3PS(GB)**

**EP 0 129 506 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue heterocyclisch substituierte Thio(cyclo)alkan-polycarbonsäuren, welche als Korrosionsinhibitoren verwendbar sind. Es handelt sich dabei um Benzthiazol--2-thiopolycarbonsäuren.

Das US-Patent 2,725,364 beschreibt Ester der Benzthiazol-2-ylthio-bernsteinsäure der Formel

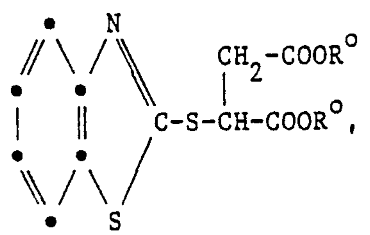

worin $R^O$ einen Kohlenwasserstoffrest oder Oxakohlenwasserstoffrest mit 3-12 C-Atomen bedeutet, und die als Weichmacher für Vinylchlorid-Polymerisate verwendet werden können. Ihre Herstellung geschieht durch Basen-katalysierte Addition von Estern der Maleinsäure an 2-Mercaptobenzthiazol. Es wird erwähnt, dass die Ester auch durch Veresterung der entsprechenden Dicarbonsäure ($R^o = H$) hergestellt werden können und dass diese Dicarbonsäure durch Addition von Malein- oder Fumarsäure an 2-Mercaptobenzthiazol in wässrig-alkalischem Medium erhalten werden können. Es werden dort jedoch keine Details der Eigenschaften und Herstellung dieser Dicarbonsäure beschrieben. Eigene Versuche zeigten, dass 2-Mercaptobenzthiazol im wässrig-alkalischen Medium mit Malein- oder Fumarsäure nicht reagiert. Weiterhin zeigten eigene Versuche, dass die auf anderem Wege hergestellte Benzthiazol-2-ylthio-bernsteinsäure im wässrig-alkalischem Medium bei 45-50 °C langsam in 2-Mercaptobenzthiazol und Fumarsäure gespalten wird.

Es wurde jedoch gefunden, dass diese Dicarbonsäure und ähnliche heterocyclische Mercaptocarbonsäuren auf andere Weise hergestellt werden können. Es handelt sich dabei um die Verbindungen der Formel I,

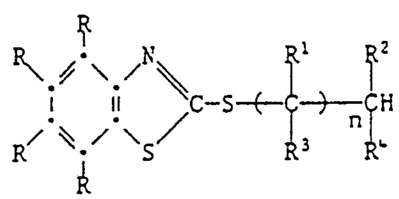

I

worin jedes R unabhängig von den anderen H, $C_1$-$C_{12}$-Alkyl, $C_1$-$C_4$-Halogenalkyl, $C_1$-$C_{12}$-Alkoxy, $C_1$-$C_{12}$-Alkylthio, $C_1$-$C_{12}$-Alkylsulfonyl, Phenyl, $C_7$-$C_{16}$-Alkylphenyl, $C_7$-$C_{16}$-Phenylalkyl, $C_5$-$C_8$-Cycloalkyl, Halogen, $-NO_2$, -CN,-COOH, -COO($C_1$-$C_4$-Alkyl), -OH oder eine primäre, sekundäre oder tertiäre Amino- oder Carbamoylgruppe mit bis zu 20 C-Atomen bedeutet,

n null oder 1 ist,

und wenn n=l ist, $R^1$ $R^2$, $R^3$ und $R^4$ unabhängig voneinander H, $C_1$-$C_{18}$-Alkyl, $C_1$-$C_4$-Hydroxyalkyl, $C_2$-$C_{12}$-Carboxyalkyl, $C_1$-$C_4$-Halogenalkyl, $C_2$-$C_{10}$-Alkoxyalkyl, -COOH, Phenyl oder Benzyl bedeuten, oder $R^1$ und $R^2$ oder $R^1$ und $R^3$ zusammen unverzweigtes oder verzweigtes $C_3$-$C_8$-Alkylen bedeuten, das durch l oder 2 Carboxylgruppen substituiert sein kann, oder $R^1$ und $R^2$ zusammen eine direkte Bindung bedeuten, und wenn n=O ist, $R^2$ und $R^4$ Carboxyl oder durch 1 oder 2 Carboxyl-` gruppen substituiertes Phenyl bedeuten, unter der Massgabe, dass die Gruppe

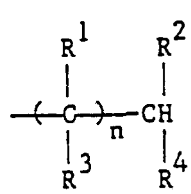

mindestens zwei und höchstens vier Carboxylgruppen enthält, sowie Basen-Additions-Salze dieser Verbindungen.

Bevorzugt sind Verbindungen der Formel I, worin n = 1 ist.

R als Alkyl kann z.B. Methyl, Ethyl, Propyl, Isopropyl, n-Butyl, Isobutyl, tert.Butyl, n-Pentyl, iso-Pentyl, n-Hexyl, 2-Ethylbutyl, n-Heptyl, n-Octyl, 1,1,3,3-Tetramethylbutyl, n-Nonyl, iso-Nonyl, n-Decyl, n-Undecyl oder n-Dodecyl sein. R als Halogenalkyl kann z.B. Chlormethyl, Trichlormethyl, Mono-, Di- oder Trifluormethyl oder 2-Chlorethyl sein. R als Alkoxy kann z.B. Methoxy, Ethoxy, Propyloxy, Isopropyloxy, Butyloxy, Hexyloxy, Octyloxy oder Dodecyloxy sein. R als Alkylthio kann z.B. Methylthio, Ethylthio, Isopropylthio, n-Butylthio, tert.Butylthio, n-Hexylthio oder n-Dodecylthio sein. R als Alkylsulfonyl kann z.B. Methylsulfonyl, tert.Butylsulfonyl, n-Octylsulfonyl, 2-Ethylhexylsulfonyl oder n-Dodecylsulfonyl sein. R als Alkylphenyl kann z.B. Tolyl, Xylyl, Ethylphenyl, tert.Butylphenyl, Hexylphenyl oder Nonylphenyl sein. R als Phenylalkyl kann z.B. Benzyl, 1-Phenylethyl, 2-Phenylethyl oder 1-Phenylbutyl sein. R als Cycloalkyl kann z.B. Cyclopentyl, Cyclohexyl oder Cyclooctyl sein. R als -COOAlkyl kann z.B. Methoxycarbonyl, Ethoxycarbonyl, Isopropoxycarbonyl oder Butoxycarbonyl sein. R als Halogen kann z.B. Fluor, Chlor oder Brom sein. R als primäre, sekundäre oder tertiäre Aminogruppe kann z.B. eine Amino, Isopropylamino-, Anilino-, tert.Butylamino-, Dimethylamino-, Diethylamino-, Dipropylamino-, Di(hydroxyethyl)amino-, Dibutylamino-, Dioctylamino-, Cyclohexylamino-, N-Methylanilino-, N-Methyl-benzylamino-, Piperidino- oder Morpholinogruppe sein. R als primäre. sekundäre oder tertiäre Carbamoylgruppe kann z.B. $-CONH_2$, $-CONHCH_3$, -CONHPhenyl, -CONHCyclohexyl, $-CON(CH_3)_2$, $-CON(C_6H_{13})_2$ oder Morpholinocarbonyl oder Piperidinocarbonyl sein.

Vorzugsweise ist einer der Substituenten R Wasserstoff, $C_1$-$C_4$-Alkyl, $C_1$-$C_4$-Alkoxy oder Amino und die anderen drei R sind H. Besonders bevorzugt sind alle vier R Wasserstoff.

$R^1$ $R^2$ $R^3$ und $R^4$ als Alkyl können z.9. Methyl, Ethyl, n-Propyl, Isopropyl, n-Butyl, Isobutyl, n-Pentyl, n-Hexyl, n-Octyl, Isooctyl, n-Decyl, n-Dodecyl, n-Hexadecyl oder n-Octadecyl sein. $R^1$, $R^2$, $R^3$, $R^4$ als Hydroxy- oder Alkoxyalkyl können z.B. Hydroxymethyl, 1-Hydroxyethyl, 2-Hydroxyethyl, 2-Hydroxypropyl, 3-Hydroxypropyl, 2-Hydroxybutyl, 2-Methoxyethyl, Isopropoxymethyl, 2-Butoxyethyl, Octyloxymethyl , 2-Ethoxypropyl oder 3-Methoxypropyl sein. $R^1$, $R^2$, $R^3$, $R^4$ als Halogenalkyl können z.B. Fluor- oder Chloralkyl sein wie z.B. Mono-, Di- oder Trifluormethyl, Chlormethyl oder 2-Chlorethyl. $R^1$, $R^2$, $R^3$, $R^4$ als Carboxyalkyl können Mono-, Di- oder Tricarboxyalkyl sein wie z.B. Carboxymethyl, 1- oder 2-Carboxyethyl, 1-, 2- oder 3-Carboxypropyl, 1-Carboxyisopropyl, 4-Carboxybutyl, 6-Carboxyhexyl, 11-Carboxyundecyl, 1,2-Dicarboxyethyl, 2,3-Dicarboxypropyl oder 2,3,4-Tricarboxybutyl.

Wenn $R^1$ und $R^2$ oder $R^1$ und $R^3$ zusammen Alkylen bedeuten, so bilden sie zusammen mit den C-Atomen, an die sie gebunden sind, einen cycloaliphatischen Ring, insbesondere einen Cyclopentan- oder Cyclohexanring, der durch Niederalkylgruppen und durch 1 oder 2 Carboxylgruppen substituiert sein kann.

Wenn $R^1$ und $R^2$ zusammen eine direkte Bindung bedeuten, so stellen die Verbindungen der Formel I ungesättigte Polycarbonsäuren dar.

Im Falle von n = O sind $R^2$ und $R^4$ Carboxyl oder Carboxyalkyl. Im Falle von n = 1 sind mindestens zwei der Substituenten $R^1$, $R^2$, $R^3$ und $R^4$ Carboxyl oder Carboxyalkyl. Vorzugsweise sind $R^1$, $R^2$, $R^3$ und $R^4$ unabhängig voneinander H, $C_1$-$C_{12}$-Alkyl, $C_1$-$C_4$-Hydroxyalkyl, $C_2$-$C_6$-Carboxyalkyl, $C_2$-$C_{10}$-Alkoxyalkyl, Carboxyl, Phenyl oder Benzyl oder $R^1$ und $R^2$ zusammen sind Trimethylen oder Tetramethylen. Besonders bevorzugt sind $R^1$, $R^2$, $R^3$ und $R^4$ Wasserstoff, $C_1$-$C_4$-Alkyl, Carboxyl oder $C_2$-$C_6$-Carboxyalkyl. Bevorzugt sind weiterhin Verbindungen, in denen zwei der Substituenten $R^1$, $R^2$, $R^3$, $R^4$ Carboxyl oder Carboxyalkyl sind.

Auf Grund dieser Definition ist die Gruppe

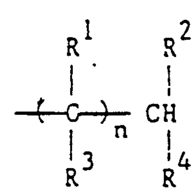

eine Di-, Tri- oder Tetracarbonsäuregruppe, vorzugsweise eine Dicarbonsäuregruppe. Beispiele hierfür sind: Dicarboxymethyl, 1,2-Dicarboxyethyl, 1,2-Dicarboxyethenyl, 1,2-Dicarboxypropyl, 2,3-Dicarboxypropyl, 1,3-Dicarboxy-2-propyl, 1,3-Dicarboxypropyl, 1,2-Dicarboxyprop-1-enyl, 2,3-Dicarboxy-1-phenylpropyl, 2,3-Dicarboxy-1, 1 -diphenylpropyl, 1,2-Dicarboxy-2-methylpropyl, 2,3-Dicarboxy-2-butyl, 1,2-Dicarboxybutyl, 2,4-Dicarboxybutyl, 3,4-Dicarboxybutyl, 1,4-Dicarboxybutyl, 3,4-Dicarboxy-3-hexyl, 1,2,3-Tricarboxypropyl, 2,3,4-Tricarboxybutyl, 1,1,2,2-Tetracarboxyethyl, 2,4,6,8-Tetracarboxyoctyl, 1,2-, 2,3- oder 3,4-Dicarboxycyclohexyl.

Die Basen-Additions-Salze dieser Verbindungen sind vorzugsweise Salze von Alkalimetallen, Erdalkalimetallen, Metallen der Gruppe IIB, IIIA oder VIII des Periodensystems oder Salze von Ammoniak oder von organischen Aminen, insbesondere Natrium-, Kalium-, Calcium-, Magnesium-, Zink-, Aluminium-, Ammoniumsalze oder Salze von primären, sekundären oder tertiären Aminen, wie z.B. von Methylamin, Ethanolamin, Diethylamin, Dibutylamin, Diethanolamin, Cyclohexylamin, Trimethylamin, Tri(isopropyl)amin, Trioctylamin, Triethanolamin oder von technischen Amin-Gemischen.

Beispiele für einzelne Verbindungen der Formel I sind: Benzthiazol-2-ylthio-malonsäure, Benzthiazol-2-ylthio-bernsteinsäure, 5-Methylbenzthiazol-2-ylthio-bernsteinsäure, 5-Trifluormethylbenzthiazol-2-ylthio-bernsteinsäure, 6-Ethylbenzthiazol-2-ylthio-bernsteinsäure, 4-Isopropylbenzthiazol-2-ylthio-bernsteinsäure, 7-t-Butylbenzthiazol-2-ylthio-bernsteinsäure, 5-n-Hexylbenzthiazol-2-ylthio-bernsteinsäure, 6[1,1,3,3-Tetramethylbutyl]-benzthiazol-2-ylthio-bernsteinsäure, 6-Cyclohexylbenzthiazol-2-ylthio-bernsteinsäure, 7-Benzylbenzthiazol-2-ylthio-bernsteinsäure, 6-Methoxybenzthiazol-2-ylthio-bernsteinsäure, 6-Ethoxybenzthiazol-2-ylthio-bernsteinsäure, 7-Ethoxybenzthiazol-2-ylthio-bernsteinsäure, 5-Methoxybenzthiazol-2-ylthio-bernsteinsäure, 4-Methylthiobenzthiazol-2-ylthio-bernsteinsäure, 4-Fluorbenzthiazol-2-ylthio-bernsteinsäure, 5-Chlorbenzthiazol-2-ylthio-bernsteinsäure, 7-Brombenzthiazol-2-ylthio-bernsteinsäure, 6-Chlorbenzthiazol-2-ylthio-bernsteinsäure, 4-Phenylbenzthiazol-2-ylthio-bernsteinsäure, 6-Nitrobenzthiazol-2-ylthio-bernsteinsäure, 5-Carboxybenzthiazol-2-ylthio-bernsteinsäure, 5-Ethoxycarbonylbenzthiazol-2-ylthio-bernsteinsäure, 7-Hydroxybenzthiazol-2-ylthio-bernsteinsäure, 5-Chlor-6-n-butylbenzthiazol-2-ylthio-bernsteinsäure, 5-Brom-5-n-hexylbenzthiazol-2-ylthio-bernsteinsäure, 5-Nitro-6-n-propylbenzthiazol-2-ylthio-bernsteinsäure, 5-Cyanobenzthiazol-2-ylthio-bernsteinsäure, 5-Brom-6-n-propoxybenzthiazol-2-ylthio-bernsteinsäure, 6-Aminobenzthiazol-2-ylthio-bernsteinsäure, 6-Methylaminobenzthiazol-2-yl-bernsteinsäure, 6-Dimethylaminobenzthiazol-2-yl-bernsteinsäure, 7-Phenylaminobenzthiazol-2-yl-bernsteinsäure, 4-Dibenzylaminobenzthiazol-2-yl-bernsteinsäure, 4-Morpholinobenzthiazol-2-yl-bernsteinsäure, 5-Carbamoylbenzthiazol-2-yl-bernsteinsäure, 5-Methylcarbamoylbenzthiazol-2-yl-bernsteinsäure, 5-Diethylcarbamoylbenzthiazol-2-yl-bernsteinsäure, 6-Phenylcarbamoylbenzthiazol-2-yl-bernsteinsäure, 5,6-Dimethyl-benzthiazol-2-ylthio-bernsteinsäure, 4,5,6-Triethylbenzthiazol-2-ylthio-bernsteinsäure, 4,5,6,7-Tetramethylbenzthiazol-2-ylthio-bernsteinsäure, 1-(Benzthiazol-2-ylthio)-propan-1 1,2-dicarbonsäure, 1-(4-Phenylbenzthiazol-2-ylthio)-propan-1 1,2-dicarbonsäure, 3-(Benzthiazol-2-ylthio)-propan-1,2-dicarbonsäure, 3-(6-Trifluormethylbenzthiazol-2-yl)-propan-1,2-dicarbonsäure, 3-(6-Methoxycarbonylbenzthiazol-2-yl)-propan-1,2-dicarbonsäure, 3-(6-Aminobenzthiazol-2-yl)-propan-1,2-dicarbonsäure, 3-(4-Morpholinobenzthiazol-2-yl)-propan-1,2-dicarbonsäure, 1-(Benzthiazol-2-ylthio)-propan-1,3-dicarbonsäure, 1-(6-Ethylthiobenzthiazol-2-ylthio)-propan-1,3-dicarbonsäure, 2-(Benzthiazol-2-ylthio)-propan-1,3-dicarbonsäure, 2-(5-Carboxybenzthiazol-2-ylthio)-propan-1,3-dicarbonsäure, 3-(Benzthiazol-2-ylthio)-3-phenylpropan-1,2-dicarbonsäure, 3-(Benzthiazol-2-ylthio)-3,3-diphenylpropan-1,2-dicarbonsäure, 1-(Benzthiazol-2-ylthio)-butan-1,2-dicarbonsäure, 1-(4-Methoxy-6-hydroxybenzthiazol-2-ylthio)-butan-1,2-dicarbonsäure, 1-Benzthiazol-2-ylthio)propan-2,3-dicarbonsäure, 1-(4,5-Dimethyl-7-propoxybenzthiazol-2-ylthio)-propan-2,3-dicarbonsäure, 1-(Benzthiazol-2-ylthio)-2-methylpropan-1,2-dicarbonsäure, 2-(Benzthiazol-2-ylthio)-butan-2,3-dicarbonsäure, 1-(Benzthiazol-2-ylthio)-butan-2,4-dicarbonsäure, 4-(benzthiazol-2-ylthio)-butan-1,2,3-tricarbonsäure, 4-(Benzthiazol-2-ylthio)-butan-1,4-dicarbonsäure, 1-(Benzthiazol-2-ylthio)-pentan-1,5-dicarbonsaure, 1-(Benzthiazol-2-ylthio)-hexan-1,6-dicarbonsäure, 4-(Benzthiazol-2-ylthio)-cyclohexan-1,2-dicarbonsäure, 1-(Benzthiazol-2-ylthio)-cyclohexan-1,2-dicarbonsäure, 1-(Benzthiazol-2-ylthio)propan-1,2,3-tricarbonsäure, 3-(Benzthiazol-2-ylthio)-3-chlorpropan-1,3-dicarbonsäure, 1-(Benzthiazol-2-ylthio)-3-methoxypropan-1,2-dicarbonsäure, 1-(Benzthiazol-2-ylthio)-3-hydroxypropan-1,2-dicarbonsäure, 1-(Benzthiazol-2-ylthio)-2-phenyl-bernsteinsäure, 1-(Benzthiazol-2-ylthio)-2-benzyl-bernsteinsäure, 1-(Benzthiazol-2-ylthio)-3-methylbutan-1,3-dicarbonsäure, 3-(Benzthiazol-2-ylthio)-hexan-3,4-dicarbonsäure,
und die Basen-Additionssalze dieser Verbindungen wie z.B. die Natrium-, Kalium-, Calcium-, Magnesium-, Zink-, Cobalt-, Aluminium-, Ammonium-, Mono-, Di- und Trialkylammonium-, Cyclohexylammonium- oder Tris-hydroxyethyl-ammonium-Salze.

Die Verbindungen der Formel I können hergestellt werden durch Umsetzung einer Verbindung der Formel II

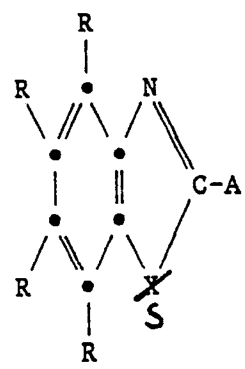

II,

worin R die vorhin gegebene Bedeutung hat und A eine Abgangsgruppe wie z.B. Cl, Br, I oder Benzol- oder p-Toluolsulfonyloxy ist, mit einer Verbindung der Formel III

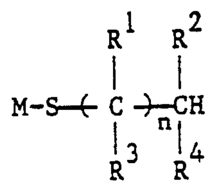

(III),

worin n, $R^1$, $R^2$, $R^3$ und $R^4$ die vorhin gegebene Bedeutung haben und M Wasserstoff oder ein Kation ist wie z.B. ein Alkalimetall-, Erdalkalimetall-oder gegebenenfalls substituiertes Ammoniumkation.

Alternativ kann eine Verbindung der Formel IV

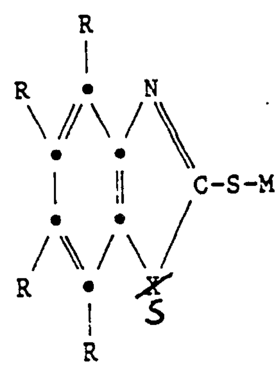

(IV)

mit einer Verbindung der Formel V

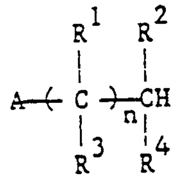

(V)

umgesetzt werden.

Diese Reaktionen können in üblicher Weise in Gegenwart einer organischen oder anorganischen Base ausgeführt werden. Beispiele für solche Basen sind tertiäre Amine, Ammoniumhydroxid, Natriummethoxid. Natrium- oder Kaliumhydroxid oder Natriumcarbonat. Wenn ein entsprechender Ueberschuss von Base verwendet wird, so fällt das Produkt der Formel I zunächst als Basen-Salz an und die freie Carbonsäure kann daraus durch Neutralisation mit einer Säure erhalten werden.

Die Umsetzungen können in einem organischen Lösungsmittel ausgeführt werden, wie z.B. in Methanol, Ethanol, Acetonitril, Toluol, Chloroform, Dimethylsulfoxid oder Dimethylformamid. Bevorzugt wird die Umsetzung im wässrig-alkalischen Medium, z.B. in wässriger $Na_2CO_3$-Lösung oder NaOH-Lösung, ausgeführt. Die Isolierung des Produktes kann dann durch Zusatz einer Mineralsäure geschehen.

Die Umsetzungen können bei Raumtemperatur oder erhöhter Temperatur ausgeführt werden, vorzugsweise arbeitet man bei 50 bis 100° C.

Gewisse Verbindungen der Formel I können in andere Verbindungen der Formel I durch bekannte Verfahren umgewandelt werden. Beispielsweise kann eine Verbindung mit einer Nitrogruppe im Benzorest durch Hydrogenierung in die entsprechende Aminoverbindung umgewandelt werden.

Beispiele von Verbindungen der Formel II sind 5-Methyl-2-brom-benzthiazol, 6-Ethyl-2-chlor-benzthiazol, 4-Isopropyl-2-iod-benzthiazol, 7-t-butyl-2-brom-benzthiazol, 5-n-Hexyl-2-brom-benzthiazol, 6-(1,1,3,3-Tetramethylbutyl)-2-chlor-benzthiazol, 6-Cyclohexyl-2-iod-benzthiazol, 7-Benzyl-2-chlor-benzthiazol, 5-Trifluormethyl-2-chlorbenzthiazol, 6-Methoxy-2-toluolsulphonyloxy-benzthiazol, 6-Ethoxy-2-chlor-benzthiazol, 7-Ethoxy-2-chlorbenzthiazol, 5-Methoxy-2-iod-benzthiazol, 5-Ethoxycarbonyl-2-brombenzthiazol, 4-Methylthio-2-brom-benzthiazol, 6-Isopropoxy-2-chlorbenzthiazol, 5-Cyano-2-chlor-benzthiazol, 4-Fluor-2-chlor-benzthiazol, 5-Chlor-2-brom-benzthiazol, 6-Chlor-2-iod-benzthiazol, 4-Phenyl-2-chlor-benzthiazol, 6-Nitro-2-chlor-benzthiazol, 6-Methylsulfonyl-2-chlor-benzthiazol, 5-Carboxy-2-iod-benzthiazol, 7-Hydroxy-2-chlor-benzthiazol, 6-Amino-2-chlor-benzthiazol, 5-Chlor-6-n-butyl-2-brom-benzthiazol, 4-Brom-5-n-hexyl-2-chlor-benzthiazol, 5-Nitro-6-n-propyl-2-chlor-benzthiazol, 5-Brom-6-n-propoxy-2-iod-benzthiazol, 4-Nitro-7-butyl-2-chlor-benzthiazol, 4,5,6-Triethyl-2-brombenzthiazol, 4,5,6,7-Tetramethyl-2-chlor-benzthiazol, 4-Methoxy-6-hydroxy-2-chlor-benzthiazol, 4,5-Dimethyl-7-propoxy-2-iod-benzthiazol, 5-Dimethylamino-2-brom-benzthiazol, 4-Morpholino-2-chlor-benzthiazol. 5-Diethylcarbamoyl-2-brom-benzthiazol.

Beispiele von Reaktanden der Formel III sind Mercaptobernsteinsäure, Trinatriumsalz der Mercaptobernsteinsäure, 2-Mercapto-3,3-dimethylbernsteinsäure, 2-oder 3-Mercaptoglutarsäure, 2-(Mercaptomethyl)-bernsteinsäure, 2-Mercapto-3-methylbernsteinsäure, 2-Mercapto-3-ethylbernsteinsäure, 2-(Mercaptomethyl)-glutarsäure, 2-Mercaptotricarballylsäure, 1-Mercapto-ethan-1,1,2,2-tetracarbonsäure, l-Mercaptocyclohexan-1,2-dicarbonsäure, 2-Mercapto-2,3-diethylbernsteinsäure, 2-Mercapto-3-chlormethyl-bernsteinsäure, 2-Mercapto-3-methoxymethyl-bernsteisäure, 2-Mercapto-3-hydroxymethyl-bernsteinsäure, 2-Mercapto-3-phenylbernsteinsäure, 2-Mercapto-3-benzylbernsteinsäure.

Beispiele von Verbindungen der Formel IV sind 5-Methyl-2-mercapto-benzthiazol, 6-Ethyl-2-mercapto-benzthiazol, 4-Isopropyl-2-mercapto-benzthiazol, 7-t-Butyl-2-mercapto-benzthiazol, 5-n-Hexyl-2-mercapto-benzthiazol, 6-(1,1,3,3-Tetramethylbutyl)-2-mercapto-benzthiazol, 6-Cyclohexyl-2-mercapto-benzthiazol, 7-Benzyl-2-mercapto-benzthiazol, 5-Trifluormethyl-2-mercapto-benzthiazol, 6-Methoxy-2-mercapto-benzthiazol, 6-Ethoxy-2-mercapto-benzthiazol, 7-Ethoxy-2-mercapto-benzthiazol, 5-Methoxy-2-mercapto-benzthiazol, 4-Methylthio-2-mercapto-benzthiazol, 6-Methylsulfonyl-2-mercapto-benzthiazol, 6-Isopropyl-2-mercapto-benzthiazol, 4-Fluor-2-mercapto-benzthiazol, 5-Chlor-2-mercapto-benzthiazol, 7-Brom-2-mercapto-benzthiazol, 6-Chlor-2-mercapto-benzthiazol, 4-(Phenyl-2-mercapto)-benzthiazol, 6-Nitro-2-mercapto-benzthiazol, 5-Cyano-2-mercapto-benzthiazol, 5-Carboxy-2-mercapto-benzthiazol, 5-Methoxycarbonyl-2-mercapto-benzthiazol, 7-Hydroxy-2-mercapto-benzthiazol, 6-Amino-2-mercapto-benzthiazol, 5-Dimethylamino-2-mercapto-benzthiazol, 5-Morpholino-2-mercapto-benzthiazol, 5-Carbamoyl-2-mercapto-benzthiazol, 5-Phenylcarbamoyl-2-mercapto-benzthiazol, 5-Chlor-6-n-butyl-2-mercapto-benzthiazol, 4-Brom-5-n-hexyl-2-mercapto-benzthiazol, 5-Nitro-6-n-propyl-2-mercapto-benzthiazol, 5-Brom-6-n-propoxy-2-mercapto-benzthiazol, 4-Nitro-7-butyl-2-mercapto-benzthiazol, 4,5,6-Triethyl-2-mercapto-benzthiazol, 4,5,6,7-Tetramethyl-2-mercapto-benzthiazol, 4-Methoxy-6-hydroxy-2-mercapto-benzthiazol, 4,5-Dimethyl-7-propoxy-2-mercapto-benzthiazol und die Salze dieser Verbindungen wie beispielsweise die Natrium-, Kalium-, Calcium-, Tetramethylammonium- oder Tris-hydroxyethyl-ammonium-Salze.

Beispiele von Reaktanden der Formel V sind Brommalonsäure, Chlorbernsteinsäure, Brombernsteinsäure, p-Toluolsulfonyloxybernsteinsaure, 2-Brom-3,3-dimethylbernsteinsäure, 2-Chlormethyl-bernsteinsäure, 2-Brommethyl-bernsteinsäure, 2- oder 3-Bromglutarsäure, 2-Chlor-3-ethylbernsteinsäure, 2-Jod-3,3-dimethylbernsteinsäure, 2-Brom-2,3-dimethylbernsteinsäure, 2-Chlormethyl-glutarsäure, 2(p-Toluolsulfonyloxymethyl)-glutarsäure, 2-Bromtricarballylsäure, 1-Jodethantetracarbonsäure, 1-Bromcyclohexan-1,2-dicarbonsäure, 2-Jod-2-butyl-bernsteinsäure, 2-Brom-3-methoxymethyl-bernsteinsäure, 2-Chlor-3-hydroxymethylbernsteinsäure, 2-Brom-3-phenylbernsteinsäure, 2-Jod-3-benzyl-bernsteinsäure, Brom-maleinsäure, 2-Brom-3-methylmaleinsäure, 2-Brom-2,3-dimethylfumarsäure.

Die Verbindungen der Formel I, worin $R^4$ Carboxyl ist, können auch nach einem anderen Verfahren hergestellt werden, das Gegenstand dev EP-A-126 030 ist. Nach diesem Verfahren wird eine Verbindung der Formel IV, worin M = H ist, in stark saurem Medium mit einer $\alpha,\beta$-ungesättigten Carbonsäure der Formel VI

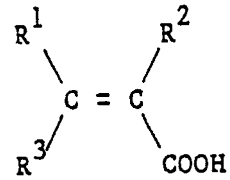 (VI)

umgesetzt. Beispiele für Verbindungen der Formel VI sind Maleinsäure, Fumarsäure, Mesaconsäure, Citraconsäure, Itaconsäure, Aconitsäure, Glutaconsäure oder Methylenglutarsäure.

Die Verbindungen der Formel I können als Korrosionsinhibitoren für Metalle, wie z.B. Eisen, Stahl, Aluminium oder Kupfer, vor allem aber für Eisenmetalle, verwendet werden. Dies betrifft vor allem ihre Verwendung in wässrigen Systemen, die mit Metallen in Kontakt sind, beispielsweise in Kühlwassersystemen, Klimaanlagen, Dampferzeugungsanlagen, Seewasserverdampfungsanlagen, Heizungs- und Kühlwasser-Kreisläufen, wässrigen Metallbearbeitungs-, Gefrierschutz-und Hydraulik-Flüssigkeiten.

In der EP-A-3817 wurden bereits Zink- und Bleisalze von 2-Mercaptobenzthiazol als Korrosionsinhibitoren für Ueberzugsmittel vorgeschlagen. In der CH-A-458 048 werden Derivate des 2-Mercaptobenzoxazols und des 2-Mercaptobenzimidazols als Korrosionsschutzmittel bei der Papierherstellung vorgeschlagen, darunter auch entsprechende Thiocarbonsäuren. α-Benzthiazolylthioalkancarbonsäuren wurden im GB-A-l 028 924 als Korrosionsinhibitoren für Schmiermittel vorgeschlagen. Benzimidazol-2-ylthiobernsteinsäuren wurden in der JP-A-75/52065 (=Chem. Abstr. 83, 206268r) als Pharmazeutika vorgeschlagen. β-(Benzthiazol-2-ylthio)-α-cyanoacrylsäurederivate wurden in der EP-A-93 908 als Schädlingsbekämpfungsmittel vorgeschlagen. Alle diese bekannten ähnlichen Verbindungen werden in ihrer Korrosionsschutzwirkung von den Verbindungen der Formel I übertroffen.

Die Erfindung betrifft daher ein Verfahren zum Korrosionsschutz von wässrigen Systemen, die in Kontakt mit Metallen stehen, durch Zugabe einer korrosionsinhibierenden Menge einer Verbindung der Formel I, wie sie eingangs definiert wurde, zum wässrigen System.

In der Praxis setzt man die Verbindung der Formel I zum wässrigen System in einer Menge von 0,1 bis 50.000 ppm, vorzugsweise 1 bis 500 ppm, zu. Ausser dem Korrosionsinhibitor können noch andere Additive, wie sie zur Wasserbehandlung bekannt sind, zugesetzt werden.

Im Falle von rein-wässerigen Systemen, wie in Kühlwassersystemen, Klimaanlagen, Dampferzeugungsanlagen, Seewasserverdampfungsanlagen, Heizungs- und Kühlwasserkreisläufen können als zusätzliche Korrosionsinhibitoren z.B. wasserlösliche Zinksalze, Phosphate, Polyphosphate, Phosphonsäuren und deren Salze, wie Acetodiphosphonsäure, Phosphonocarboxylsäuren und deren Salze, wie z.B. Nitrilotris-(methylenphosphonsäure), die Methylaminodimethylenphosphonocarbonsäuren der DE-OS 26 32 774, Hydroxyphosphonoessigsäure, 2-Phosphonobutan-l,2,4-tricarbonsäure und die Säuren der GB 1,572,406, Chromate wie Natriumchromat, Nitrate wie Natriumnitrat, Nitrite wie $NaNO_2$, Molybdate wie Natrium-Molybdat, Wolframate, Silicate, Benztriazole und Benztriazolderivate, N-Acylsarkosine, N-Acylamino-diessigsäuren, Ethanolamine, Fettamine, Polycarbonsäuren wie z.B. Polymalein- oder Polyacrylsäure und deren Salze, Copolymerisate von Maleinsäure und von Acrylsäure sowie Substitutionsderivate solcher (Co)-polymerisate verwendet werden.

Dazu können noch Dispergierhilfsmittel zugesetzt werden wie z.B. Polyacrylsäure und deren Salze, Phosphinopolycarbonsäuren (wie im GB 1,458,235 beschrieben), hydrolysiertes Polyacrylnitril, Polymethacrylsäure und deren Salze, Polyacrylamid und dessen Copolymere mit Acryl- oder Methacrylsäure, Ligninsulfonsäure und ihre Salze, Taurin, Naphthalinsulfonsäure-Formaldehyd-Kondensate, Stärke und deren Derivate sowie Cellulosederivate. Spezifische Beispiele hierfür sind 2-Phosphonobutan-1,2,4-tricarbonsäure, Acetodiphosphonsäure, hydrolysiertes Polymaleinsäureanhydrid und Salze davon, Alkylphosphonsäuren, Hydroxyphosphonessigsäure, 1-Aminoalkyl-1,1-diphosphonsäuren und deren Salze und Alkalipolyphosphonate.

Zugesetzt werden können auch Fällungsmittel wie Alkali-orthophosphate oder Alkalicarbonate; Sauerstoff-Abfänger wie Alkalisulfite und Hydrazine; Komplexierungsmittel wie Nitrilotriessigsäure und deren Salze; Schaumdämpfer wie Silikone, Dicarbonsäure-fettamide, Ethylenoxid-Addukte von Fettalkoholen oder Fettamiden; Desinfektionsmittel wie Amine, quaternäre Ammoniumsalze, Chlorphenole, Sulfone, Thiocyanate, Carbamate, Isothiazolone, bromierte Propionamide, Triazine, Phosphoniumsalze, Chlor und Chlorabgebende Verbindungen oder Organozinnverbindungen.

Das wässrige System kann auch partiell wässrig dein wie z.B. in wasserverdünnbaren Metallbearbeitungsflüssigkeiten. Solche Formulierungen können z.B. verwendet werden zum Polieren, Bohren, Fräsen, Schneiden, Sägen, Ziehen oder Bördeln von Metallen. Von ihrer Formulierung her mögen folgende Typen

genannt werden:

a) Wässrige Konzentrate eines oder mehrerer Korrosionsinhibitoren und gegebenenfalls eines oder mehrerer Antiverschleiss-Additive, die in einer Verdünnung von 1:50 bis 1:100 als Schleif-Flüssigkeiten verwendet werden.

b) Polyglykole enthaltend Biocide, Korrosioinsinhibitoren und Antiverschleiss-Additive, welche in einer Verdünnung von 1:20 bis 1:40 als Schneidöl und in einer Verdünnung von 1:60 bis 1:80 als Schleiföl verwendet werden.

c) Halbsynthetische Schneidöle ähnlich b), die ausserdem noch 10-25% eines Oeles enthalten sowie eine entsprechende Menge Emulgator, um bei Verdünnung mit Wasser durchscheinend zu bleiben.

d) Ein emulgierbares Mineralölkonzentrat, das z.B. Emulgatoren, Korrosionsinhibitoren, Hochdruck-Additive, Biocide, Schaumdämpfungsmittel u.a. enthalten kann. Diese werden im allgemeinen im Verhältnis 1:10 bis 1:50 mit Wasser verdünnt, wobei sie eine weiss-opake Emulsion bilden.

e) Mineralölkonzentrate ähnlich d), die jedoch weniger Oel und mehr Emulgator enthalten und bei einer Verdünnung von 1:50 bis 1:100 eine durchscheinende Emulsion ergeben und die als Schneid- und Schleiföle verwendet werden.

Für solche partiell wässrige Systeme, wie sie als Metallbearbeitungsformulierungen verwendet werden, kann der erfindungsgemässe Korrosionsinhibitor der Formel I allein oder im Gemisch mit anderen Korrosionsinhibitoren oder sonstigen Additiven verwendet werden. Beispiele für zusätzliche Korrosionsinhibitoren sind organische Säuren und deren Ester und Salze, z.B. Benzoesäure, p-tert.Butylbenzoesäure, Natriumsebacat, Triethanolamin-laurat, Isononansäure, Triethanolaminsalz von p-Toluolsulfonamidocapronsäure, Triethanolaminsalze von 5-Ketocarbonsäuren der EP-A-41927, Natriumsalze von N-Lauroylsarcosin oder Phenoxyessigsäure; stickstoffhaltige Verbindungen wie z.B. Fettsäurealkanolamide, Imidazoline, Oxazoline, Triazole, insbesondere Benztriazole und davon abgeleitete Mannich-Basen, Trialkanolamine, Fettamine, anorganische Nitrate oder Nitrite oder die Carboxytriazine der EP-A-46139; phosphorhaltige Verbindungen wie Aminphosphate, Phosphonsäuren und deren Salze, z.B. Natriumdihydrogenphosphat oder Zinkphosphat; schwefelhaltige Verbindungen wie z.B. Na-, Ca- oder Ba-Petrolsulfonate oder das Natriumsalz von Mercaptobenzthiazol.

Als weitere Additive können Hochdruck-Additive mitverwendet werden. Dies sind meist schwefel-, phosphor- oder halogenhaltige Verbindungen, z.B. sulfuriertes Spermöl, sulfurierte Fette, Tritolylphosphat, Chlorparaffine oder ethoxylierte Phosphorester.

Wichtig für die Verwendung der Verbindungen der Formel I in wässrigen Systemen ist ihre gute Verträglichkeit mit hartem Wasser. Diese beruht auf der entsprechend hohen Löslichkeit ihrer Erdalkalisalze.

Die Verbindungen der Formel I eignen sich weiterhin auch als Korrosionsinhibitoren in Anstrichstoffen, beispielsweise in solchen auf der Basis von Epoxidharzen, Polyurethanen, Aminoplasten, Acrylharzen, Polyestern und Alkydharzen. Weitere Beispiele sind Anstrichstoffe auf Basis von Polyvinylbutyral, Phenolharzen, Polyvinylacetat, Polyvinylchlorid, Chlorkautschuk, Styrol-Butadien-Copolymeren, trocknenden Oelen oder Celluloseestern. Die Anstrichstoffe können hierbei ein wässriges System sein oder eine organische Lösung oder lösungsmittelfrei. Solche Systeme sind in der EP-A-128 862 näher beschrieben.

Die folgenden Beispiele illustrieren die vorliegende Erfindung näher. Darin bedeuten Teile und Prozente Gewichtsteile und Gewichts-Prozente, sofern nicht anders angegeben. Die Temperaturen sind in °C angegeben.

Beispiel 1: Eine Lösung von 39,4 g Brombernsteinsäure in 200 ml einer 10%igen wässrigen $Na_2CO_3$-Lösung wird innerhalb 30 Minuten unter Rühren zu einer Lösung von 41,5 g 2-Mercaptobenzthiazol in 200 ml einer 10%igen $Na_2CO_3$-Lösung bei 80° zugegeben. Die resultierende Lösung wird weitere 2 Stunden bei 80° gerührt. Es entsteht eine Dispersion, die nach Kühlen auf Raumtemperatur mit Chloroform extrahiert wird, um nicht-umgesetztes Mercaptobenzthiazol zu entfernen. Die wässrige Lösung wird dann mit konzentrierter Salzsäure angesäuert und mit Diethylether extrahiert. Die Etherlösung wird eingedampft und der feste Rückstand aus wässrigem Methanol umkristallisiert. Man erhält so die Benzthiazol-2-ylthiobernsteinsäure, die bei 175-178° (unter Zersetzung) schmilzt. NMR-Spektrum ($\delta$, DMSO - $d_6$): 3,15 (d, 2H); 4,95 (t, 1H); 7,20-8,20 (c, 4H).

Beispiel 2: Eine Lösung von 19,7 g Brombernsteinsäure in 100 ml einer 10%igen $Na_2CO_3$-Lösung wird innerhalb 30 Minuten zu einer Lösung von 20,2 g 5-Chlor-2-mercaptobenzthiazol und 4 g NaOH in 100 ml Wasser unter Rühren zugegeben. Die resultierende Suspension wird 2 Stunden auf 90° erwärmt und nach dem Erkalten filtriert. Das Filtrat wird mit konzentrierter Salzsäure auf pH 1 angesäuert und das dabei ausgefällte Produkt abfiltriert und aus wässrigem Ethanol umkristallisiert. Man erhält so die 5-Chlorbenzthiazol-2-ylthio-bernsteinsäure, die (unter Zersetzung) bei 168-173° schmilzt.

| | | | | |
|---|---|---|---|---|
| Elementaranalyse | ber. | 41,58% C | 2,53% H | 4,40% N |
| $C_{11}H_8ClNO_4S_2$ | gef. | 41,78% C | 2,63% H | 5,68% N |

In analoger Weise werden hergestellt:

6-Nitrobenzthiazol-2-ylthio-bernsteinsäure, Smp. 115-118° (Zer.)

| | | | | |
|---|---|---|---|---|
| Analyse ($C_{11}H_8N_2O_6S_2$) | ber. | 40,20% C | 2,46% H | 8,54% N |
| | gef. | 40,44% C | 2,65% H | 8,82% N |

6-Ethoxybenzthiazol-2-ylthio-bernsteinsäure, Smp. 168-170°

| | | | | |
|---|---|---|---|---|
| Analyse ($C_{13}H_{13}NO_5S_2$) | ber. | 47,72% C | 4,16% H | 4,44% N |
| | gef. | 47,40% C | 4,15% H | 4,15% N. |

6-Aminobenzthiazol-2-ylthio-bernsteinsäure, Smp. 115° (Zers.)

| | | | | |
|---|---|---|---|---|
| Analyse ($C_{11}H_{10}N_2O_4S_2$) | ber. | 44,28% C | 3,38% H | 9,39% N |
| | gef. | 44,01% C | 3,59% H | 9,31% N. |

Beispiel 3 : 4,06 g 2-Brom-3,3-dimethylbernsteinsäureanhydrid und 2,12 g $Na_2CO_3$ werden in 50 ml Wasser und bei 60° eine Stunde gerührt. Die entstandene Lösung wird auf Raumtemperatur gekühlt und tropfenweise zu einer Lösung von 3,67 g 2-Mercaptobenzthiazol und 8 g NaOH in 50 ml Wasser zugegeben. Nach der Zugabe wird das Reaktionsgemisch 2 Std. auf 80° erwärmt. Nach dem Abkühlen wird konzentrierte Salzsäure bis zu einem pH von 1 zugegeben und das ausgefallene Produkt abfiltriert. Zur Reinigung wird das Produkt in $Na_2CO_3$-Lösung gelöst und nochmals mit HC1 gefällt. Man erhält so die 2-(Benzthiazol-2-ylthio)-3,3-dimethylbernsteinsäure (= 1-(Benzthiazol-2-ylthio)-2-methylpropan-1,2-dicarbonsäure) vom Smp. 176-178°.

Beispiel 4 : In 40 ml 70%iger Schwefelsäure werden 16,8 g fein gepulvertes 2-Mercaptobenzthiazol suspendiert und 10,3 g gepulvertes Maleinsäureanhydrid innerhalb 1 h unter Rühren bei 48-51° zugegeben. Nach einer weiteren Stunde bei 50° wird die Reaktionsmischung auf Raumtemperatur gekühlt und durch tropfenweise Zugabe von 250 ml Wasser bei 25-35° verdünnt. Nach 1 h wird das ausgefallene Produkt abfiltriert und in verdünnter Natronlauge gelöst. Die Lösung wird filtriert und mit Salzsäure angesäuert. Der Niederschlag wird abfiltriert und im Vakuum bei 60° getrocknet. Man erhält so 24,7 g (87% d.Th.) Benzthiazol-2-ylthiobernsteinsäure, die bei 175-176° unter Zersetzung schmilzt.

| | | | | | |
|---|---|---|---|---|---|
| Analyse ($C_{11}H_9NO_4S_2$) | ber. | 46,64% C | 3,18% H | 4,94% N | 22,61% S |
| | gef. | 46,6% C | 3,4% H | 5,0% N | 22,5% S. |

Wird dieselbe Umsetzung mit 12,1 g Maleinsäure anstelle des Anhydrids ausgeführt, so erhält man 22,6 g (80% d.Th.) an Benzthiazolylthiobernsteinsäure.

Wird dieselbe Umsetzung mit 12,1 g Fumarsäure ausgeführt und lässt man die Komponente 12 h bei 40° reagieren, so erhält man 21,1 g (75% d.Th.) an Benzthiazolylthiobernsteinsäure.

Beispiel 5 : 8,6 g 6-Chlor-2-mercapto-4-methylbenzthiazol werden wie in Beispiel 5 beschrieben mit 4,4 g Maleinsäureanhydrid in 70%iger $H_2SO_4$ bei 47-50° umgesetzt. Das Rohprodukt wird durch Umfällen aus $NaHCO_3$-Lösung gereinigt. Man erhält 6-Chlor-4-methylbenzthiazol-2-ylthiobernsteinsäure, die bei 168-171° unter Zersetzung schmilzt.

| | | | | |
|---|---|---|---|---|
| Analyse ($C_{12}H_{10}ClNO_4S_2$) | ber. | 43,44% C | 3.04% H | 4,22% N |
| | gef. | 43,48% C | 3,20% H | 4,17% N. |

In analoger Weise erhält man aus 20 g 5-Carboxy-2-mercaptobenzthiazol und 10,26 g Maleinsäureanhydrid die 5-Carboxybenzthiazol-2-ylthiobernsteinsäure, die bei 210-215° unter Zersetzung schmilzt.

| | | | | |
|---|---|---|---|---|
| Analyse ($C_{12}H_9NO_6S_2$) | ber. | 44,04% C | 2,78% H | 4,28% N |
| | gef. | 43,9% C | 2,78% H | 4,39% N. |

Beispiel 6 : In 50 ml 70%iger Schwefelsäure werden 16,8 g gepulvertes 2-Mercaptobenzthiazol suspendiert und 13,7 g Itaconsäure werden innerhalb 30 Minuten bei 40-44° in diese Suspension eingerührt. Nach weiteren 1,5 h bei 40-44° wird die Reaktionsmischung auf Raumtemperatur gekühlt und mit Wasser verdünnt, wobei man die Temperatur unterhalb 35° hält. Die ausgefallene 3-(Benzthiazol-2-ylthio)-propan-1,2-dicarbonsäure wird abfiltriert, mit kaltem Wasser gewaschen und bei 60° im Vakuum getrocknet. Ausbeute 27,5 g (92% d.Th.), Smp. 160-166°.

| | | | | | |
|---|---|---|---|---|---|
| Analyse ($C_{12}H_{11}NO_4S_2$) | ber. | 48,48% C | 3,73% H | 4,71% N | 21,57% S |
| | gef. | 48,2% C | 3,7% H | 4,6% N | 21,3% S. |

In analoger Weise erhält man aus 84,2 g 6-Nitro-2-mercaptobenzthiazol und 52.4 g Itaconsäure die 3-(6-Nitrobenzthiazol-2-ylthio)-propan-1,2-dicarbonsäure, die bei 190-198° unter Zersetzung schmilzt.

Beispiel 7 : In 150 ml 75%iger $H_2SO_4$ werden 20,9 g 2-Methylenglutarsäure unter Rühren bei 50° gelöst und langsam 24,2 g 2-Mercaptobenzthiazol zugegeben. Die Reaktion ist exotherm und die Temperatur steigt bis 60°. Anschliessend wird das Reaktionsgemisch 2 h bei 55° gerührt. Die erhaltene braune Lösung wird in 500 ml Wasser gegossen und 1 h gerührt. Dann werden 600 ml Wasser zugegeben und der weisse Niederschlag abfiltriert. Nach Umkristallisation aus wässrigem Methanol erhält man 4-(Benzthiazol-2-ylthio)-butan-1,3-dicarbonsäure, die bei 152-154° schmilzt.

| | | | | |
|---|---|---|---|---|
| Analyse ($C_{13}H_{13}NO_4S_2$) | ber. | 50,14% C | 4,21% H | 4,50% N |
| | gef. | 50,39% C | 4,35% H | 4,36% N. |

Beispiel 8 : In 50 ml 70%iger $H_2SO_4$ werden 16,8 g 2-Mercaptobenzthiazol suspendiert und 13,7 g Glutaconsäure werden innerhalb 30 Minuten bei 45-50° unter Rühren zugegeben. Das Reaktionsgemisch wird weitere 1,5 h bei 45-50° gerührt und das Produkt wie in Beispiel 5 beschrieben isoliert. Man erhält 26 g (88% d.Th.) 3-(Benzthiazol-2-ylthio)-glutarsäure, die bei 153-154° schmilzt.

| | | | | | |
|---|---|---|---|---|---|
| Analyse $C_{12}H_{11}NO_4S_2$) | ber. | 48,48% C | 3,71% H | 4,71% N | 21,57% S |
| | gef. | 48,5% C | 3,8% H | 4,8% N | 21,2% S. |

Beispiel 9 : 4,45 g 2-Mercaptobenzthiazol und 5 g But-3-en-1,2,3-tricarbonsäure werden im Mörser gemeinsam verrieben. Das gepulverte Gemisch wird portionsweise unter Rühren in 60 ml 70%ige $H_2SO_4$ bei 48-50° innerhalb einer Stunde eingetragen. Nach weiteren 3 h bei 48-50° kühlt man das Reaktionsgemisch auf Raumtemperatur und verdünnt unter Rühren mit 250 ml Wasser bei 25-30° Die wässrige Lösung wird von etwas klebrigem Bodensatz abgegossen und mit weiteren 200 ml Wasser verdünnt. Nach 1 h Rühren wird der Niederschlag abfiltriert und getrocknet. Die erhaltene 4-(Benzthiazol-2-ylthio)-butan-1,2,3-tricarbonsäure schmilzt nach Umkristallisation aus Methanol/Wasser bei 188-190°.

| | | | | |
|---|---|---|---|---|
| Analyse ($C_{13}H_{14}NO_6S_2$) | ber. | 47,32% C | 3,69% H | 3,74% N |
| | gef. | 47,40% C | 3,86% H | 3,89% N. |

Beispiel 10 : 8,56 g Benzthiazol-2-ylthiobernsteinsäure werden in 60 ml In NaOH-Lösung unter Rühren gelöst. Eine Lösung von 3,33 g $CaCl_2$ in wenig Wasser wird tropfenweise zugegeben. Der entstandene Niederschlag von Calcium-benzthiazol-2-ylthiosuccinat wird abfiltriert, mit wenig kaltem Wasser gewaschen

und im Vakuum bei 100° getrocknet.

Analyse ($C_{11}H_7CaNO_4S_2$) ber. 41,12% C 2,20% H 4,36% N 12,47% Ca
gef. 40,69% C 2,36% H 4,23% N 12,40% Ca.

In analoger Weise wird aus 8,56 g Benzthiazol-2-ylbernsteinsäure in 100 ml Methanol und 6,58 g Zinkacetat in 70 ml Methanol das entsprechende Zinksalz gefällt, das als Monohydrat anfällt.

Analyse ($C_{11}H_7NO_4S_2Zn{\bullet}H_2O$) ber. 36,23% C 2,49% H 3,84% N 17,56% S
gef. 36,54% C 2,46% H 3,79% N 17,68% S

Beispiel 11 : Eine Lösung von 8,26 g Mercaptobernsteinsäure in 50 ml 10%iger $Na_2CO_3$-Lösung wird zu einer Lösung von 8,48 g 2-Chlorbenzthiazol in 50 ml Dioxan unter Rühren zugetropft. Nach Beendigung der Zugabe wird das Reaktionsgemisch 4 h bei 85° gerührt und dann gekühlt.

Eine Lösung von 4 g NaOH in 20 ml Wasser wird dann zugegeben und das Reaktionsgemisch weitere 5 h bei 85° gerührt. Nach dem Abkühlen wird die Lösung mit konzentrierter Salzsäure angesäuert und dreimal mit 30 ml Ethylacetat extrahiert. Die vereinigten Extrakte werden über $Na_2SO_4$ getrocknet und eingedampft. Der feste Rückstand wird mit Hexan-Diethylether 1:1 gewaschen und in $NaHCO_3$-Lösung aufgenommen. Die Lösung wird vom Ungelösten abfiltriert, mit Chloroform gewaschen und mit Salzsäure angesäuert. Das dabei ausfallende Produkt wird mit Ethylacetat extrahiert. Das durch Eindampfen der Ethylacetat-Lösung ehaltene Prodkt wird aus Wasser umkristallisiert und man erhält so die Benzthiazol-2-ylthiobernsteinsäure, die mit der gemäss Beispiel 1 hergestellten Verbindung identisch ist.

Beispiel 12 : Eine Lösung von 15,0 g 6-Nitrobenzthiazol-2-ylthiobernsteinsäure (hergestellt gemäss Beispiel 2) in 70 ml wässrigem Ammionak (d 0,95) wird unter Kühlung mit einem Eisbad bei 25-30° mit Schwefelwasserstoff gesättigt. Die tief-rote Lösung wird innerhalb einer Stunde auf 90° erwärmt und 1 h bei dieser Temperatur gehalten. Die entstandene Suspension wird gekühlt und mit Essigsäure auf pH 5 gestellt, wobei durch Kühlung die Temperatur unter 35° gehalten wird.

Der Niederschlag wird rasch abfiltriert und mit wenig Wasser gewaschen. Das Filtrat wird bei Raumtemperatur mehrere Stunden gerührt, wobei das Produkt langsam auskristallisiert. Die Kristalle werden abfiltriert und aus Methanol umkristallisiert. Man erhält so die 6-Aminobenzthiazol-2-ylthio-bernsteinsäure, Smp. 117°, welche identisch ist mit der gemäss Beispiel 2 erhaltenen Verbindung.

In analoger Wiese erhält man durch eduktion von 3-(6-Nitrobenzthiazol-2-ylthio)-propan-1,2-dicarbonsäure (erhalten gemäss Beispiel 7) die 3-(6-Aminobenzthiazol-2-ylthio)-propan-1,2-dicarbonsäure, die bei 182-185° unter Zersetzung schmilzt.

Analyse ($C_{12}H_{12}N_2O_4S_2$) ber. 46,15% C 3,88% H 8,97% N
gef. 46,38% C 4,23% H 9,01% N.

Beispiel 13 : Die korrosionsinhibierende Wirkung der erfindungsgemässen Verbindungen wird im folgenden durch einen Test mit belüftetem Wasser demonstriert. Für diesen Test wird ein Wasser mit standardisierter Korrosivität verwendet, das durch Lösen von 20 g $CaSO_4{\cdot}2H_2O$, 15 g $MgSO_4{\cdot}7H_2O$, 4,6 g $NaHCO_3$ und 7,7 g $CaCl_2{\cdot}6H_2O$ in 166 1 (45 gal) destilliertem Wasser hergestellt wird. Stahlbleche (aus weichem Fluss-Stahl) von 5 x 2,5 cm werden mit Bimssteinpulver gescheuert, eine Minute in Salzsäure getaucht, gespült, getrocknet und gewogen. Jeweils 10 mg des Korrosionsinhibitors werden in 100 ml des Standard-Wassers gelöst, was einer Konzentration von 100 ppm entspricht. Das Blech wird in die Lösung gehängt und das Gefäss bei 40° thermostatisiert. Durch die Lösung wird Luft geleitet in einer Menge von 500 ml/Minute. Das dabei verdampfende Wasser wird ständig durch destilliertes Wasser ersetzt, so dass der Flüssigkeitsspiegel im Gefäss konstant bleibt.

Nach 48 h wird das Blech herausgenommen, mit Bimsstein gescheuert, eine Minute in Salzsäure getaucht, die durch Zusatz von 1% Hexamethylentetramin inhibiert wurde, und dann gespült, getrocknet und gewogen. Eine Blindprobe ohne Korrosionsinhibitor läuft bei jedem Test mit. Die Korrosionsgeschwindigkeit (KG) berechnet sich aus dem Gewichtsverlust in mg pro $dm^2$ Metalloberfläche pro Tag Versuchsdauer. Die prozentuelle Korrosionsinhibierung ergibt sich aus der Differenz von Blindprobe und Probe nach

der Formel

$$\text{Prozentuelle Korrosionsinhibierung} = \frac{\text{KG Blindprobe-KG Probe}}{\text{KG Blindprobe}} \times 100$$

Die Ergebnisse sind in Tabelle 1 aufgeführt.

Tabelle 1

| Korrosionsinhibitor (100 ppm) | Prozentuelle Korrosionsinhibierung |
|---|---|
| 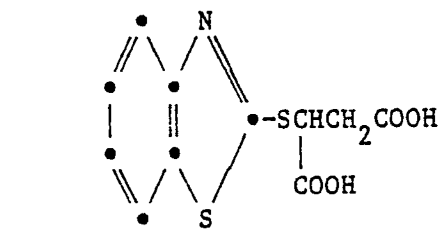 | 100 |
| 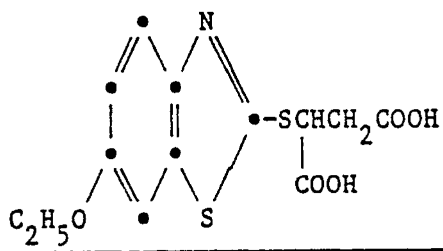 | 99 |
| 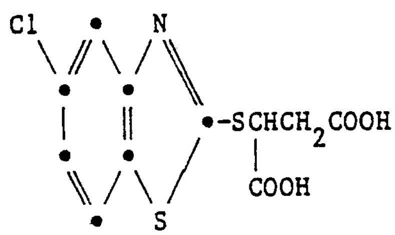 | 100 |
| 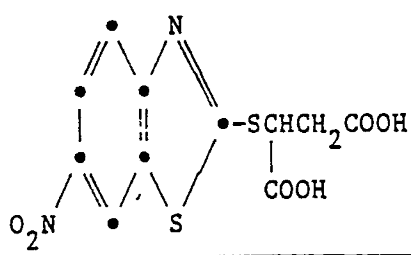 | 89 |

Beispiel 14 : Die Wirksamkeit der Korrosionsinhibitoren der Formel I in Metallbearbeitungsflüssigkeiten kann durch die Methode IP 287 des Institute of Petroleum demonstriert werden. Hierfür wird eine 1%ige Lösung des Korrosionsinhibitors in Wasser, das pro Liter 68 mg $Ca_2Cl \cdot 6H_2O$ und 132 mg $MgSO_4 \cdot 7H_2O$ enthält, durch Zusatz von Triethanolamin auf einen pH von 8,5 gebracht.

Der Boden einer Petrischale von 90 mm Durchmesser wird mit Filterpapier bedeckt und dieses auf einer Fläche von 35x35 mm mit einer dünnen Schicht von Gusseisen-Drehspänen (die nach Methode IP 287 bereitet wurden) bedeckt. Eine Menge von 2 ml der Inhibitorlösung werden auf die Drehspäne pipettiert, der Deckel der Petrischale aufgesetzt und diese 2 h bei Raumtemperatur stehen gelassen. Anschliessend wird das Filterpapier entnommen, die Späne mit Wasser abgespült und das Rostbild auf

dem Filterpapier bewertet. Folgende Beurteilungs-Skala wird in der Tabelle 2 benutzt:

0 keine Rostflecken

1 1-3 Flecken, kleiner als 1 mm Durchmesser

2 bis zu 1 der Fläche mit Flecken bedeckt

3 bis zu 5% der Fläche mit Flecken bedeckt

4 > 5% der Fläche mit Flecken bedeckt

Der Test wird auch mit einer 0,5%igen Inhibitorlösung durchgeführt. Die Ergebnisse sind in Tabelle 2 aufgeführt.

Tabelle 2

| Korrosionsinhibitoren | Rostbild bei einer Konzentration von 1% | 0,5% |
|---|---|---|
| 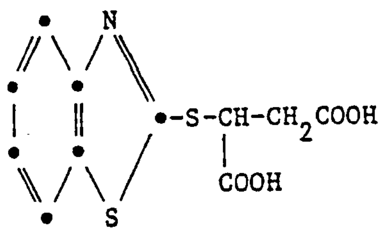 | 0 | 3 |
| 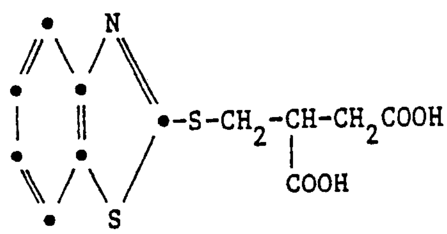 | 0 | 3 |
| 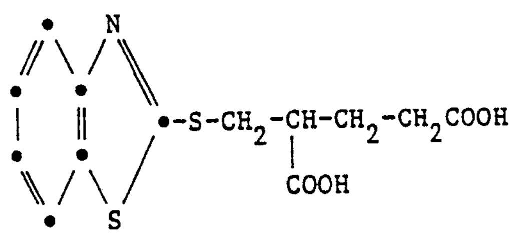 | 0 | 0 |
| 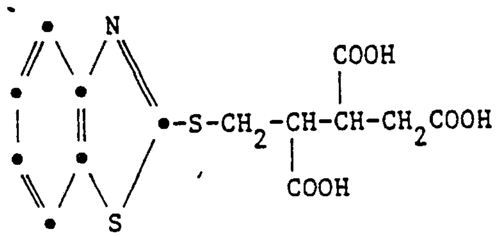 | 0 | 0 |
| ohne Inhibitor | 4 | 4 |

Visuelle Beurteilung der 1%igen Lösungen der in der Tabelle aufgeführten Verbindungen zeigen, dass innerhalb eines Tages keine Fällung oder Trübung zu erkennen ist. Dies zeigt, dass diese Verbindungen gegen Wasserhärte nicht empfindlich sind.

Beispiel 15 : Ein Primer aus einem aromatischen Epoxidharz, rotem Eisenoxid, Talkum und einem Polyaminoamid-Härter wird auf sandgestrahlte Stahlbleche aufgespritzt und 1 Woche bei Raumtemperatur

gehärtet. Darauf wird ein weisser Decklack aus einem Zweikomponenten-Polyurethan aufgetragen und ebenfalls eine Woche härten gelassen. Der Primer enthält den in der Tabelle 3 angegebenen Korrosionsinhibitor. In die lackierte Probe wird mit einem Sikkens-Messer Typ 463 ein X-förmiger Schnitt eingeschnitten, der bis in das Metall reicht. Dann werden die Bleche einem Salzsprühtest nach ASTM B 117 mit einer Dauer von 1000 Stunden unterworfen. Die Ergebnisse sind in Tabelle 3 aufgeführt.

Tabelle 3

| Korrosionsinhibitor | Blasenbildung | Unterrostung |
|---|---|---|
| keiner | stark | stark |
| 3% Verbindung 1 | wenig | leicht |
| 6% Verbindung 1 | keine | keine |

Verbindung 1 = Benzthiazol-2-ylthiobernsteinsäure

## Ansprüche

1. Verbindungen der Formel I,

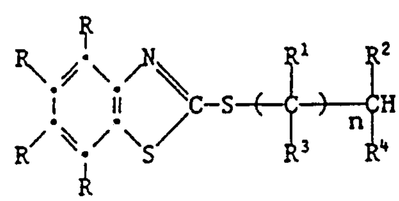

I

worin jedes R unabhängig von den anderen H, $C_1$-$C_{12}$-Alkyl, $C_1$-$C_{12}$-Alkoxy, $C_1$-$C_4$Halogenalkyl, $C_1$-$C_{12}$-Alkylthio, $C_1$-$C_{12}$-Alkylsulfonyl, Phenyl, $C_7$-$C_{16}$-Alkylphenyl, $C_7$-$C_{16}$-Phenylalkyl, $C_5$-$C_8$-Cycloalkyl, Halogen, -$NO_2$, -CN, -COOH, -COO($C_1$-$C_4$-Alkyl), -OH oder eine primäre, sekundäre oder tertiäre Amino- oder Carbamoylgruppe mit bis zu 20 C-Atomen bedeutet, n null oder 1 ist, und wenn n = 1 ist, $R^1$, $R^2$, $R^3$ und $R^4$ unabhängig voneinander H, $C_1$-$C_{18}$-Alkyl, $C_1$-$C_4$-Hydroxyalkyl, $C_2$-$C_{12}$- -Carboxyalkyl, $C_1$-$C_4$-Halogenalkyl, $C_2$-$C_{10}$-Alkoxyalkyl, -COOH, Phenyl oder Benzyl bedeuten, oder $R^1$ und $R^2$ oder $R^1$ und $R^3$ zusammen unverzweigtes oder verzweigtes $C_3$-$C_8$-Alkylen bedeuten, das durch 1 oder 2 Carboxylgruppen substituiert sein kann, oder $R^1$ und $R^2$ zusammen eine direkte Bindung bedeuten, und wenn n=0, $R^2$ und $R^4$ Carboyl oder durch 1 oder 2 Carboxylgruppen substituiertes Phenyl bedeuten, unter der Massgabe, dass die Gruppe

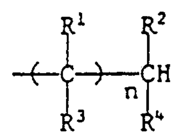

mindestens zwei und höchstens vier Carboxylgruppen enthält, sowie Basen-Additions-Salze dieser Verbindungen.

2. Verbindungen der Formel I gemäss Anspruch 1, worin ein R Wasserstoff, $C_1$-$C_4$-Alkyl, $C_1$-$C_4$-Alkoxy oder Amino ist und die anderen drei R Wasserstoff sind.

3. Verbindungen gemäss Anspruchs 2, worin alle vier R Wasserstoff sind.

4. Verbindungen der Formel I gemäss Anspruch 1, worin zwei der Gruppen $R^1$ $R^2$ $R^3$ und $R^4$ Carboxyl- oder Carboxyalkylgruppen sind. 1

5. Verbindungen der Formel I gemäss Anspruch 1, worin n = 1 ist und $R^1$, R2 $R^3$ und $R^4$ unabhängig voneinander H, $C_1$-$C_{12}$-Alkyl, $C_1$-$C_4$-Hydroxy-alkyl, $C_2$-$C_6$-Carboxyalkyl, $C_2$-$C_{10}$-Alkoxyalkyl, Carboxyl, Phenyl oder Benzyl sind, oder $R^1$ und $R^2$ zusammen Trimethylen oder Tetramethylen sind.

6. Verbindungen der Formel I gemäss Anspruchs 5, worin n = 1 ist und $R^1$, $R^2$ $R^3$ und $R^4$ unabhängig voneinander H, $C_1$-$C_4$-Alkyl, Carboxyl oder $C_2$-$C_6$--Carboxyalkyl sind.

7. Salze von Verbindungen der Formel I gemäss Anspruch 1, welche Salze eines Alkalimetalls, Erdalkalimetalls, Metalls der Gruppe 11B, 111A oder VIII des Periodensystems, von Ammoniak oder von organischen Aminen sind.

8. Salze gemäss Anspruch 7 welche Natrium-, Kalium-, Ammonium-, Calcium-. Magnesium-, Zink- oder Aluminiumsalze oder Salze von primären, sekundären oder tertiären Aminen sind.

9. Eine Verbindung gemäss Anspruch 1 der Formeln

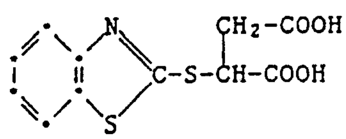 , 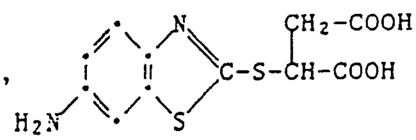

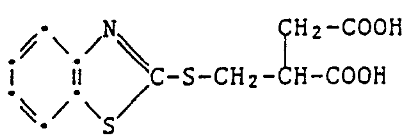 oder

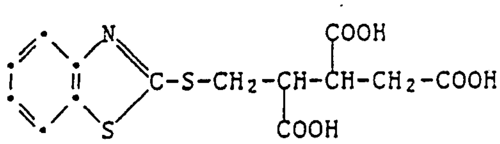 .

10. Verfahren zur Herstellung von Verbindungen der Formel I des Anspruchs 1. dadurch gekennzeichnet. dass man entweder eine Verbindung der Formel II

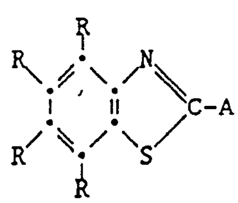 II,

worin R die im Anspruch 1 gegebene Bedeutung hat und A eine Abgangsgruppe ist, mit einer Verbindung der Formel III

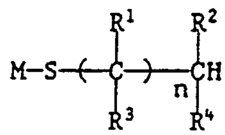

(III),

worin n, $R^1$, $R^2$, $R^3$ und $R^4$ die in Anspruch 1 gegebene Bedeutung haben und M Wasserstoff oder ein Kation ist, umsetzt oder eine Verbindung der Formel IV

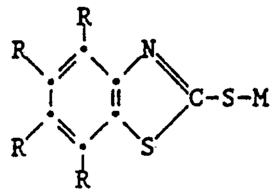

(IV)

mit einer Verbindung der Formel V

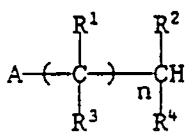

umsetzt.

11. Verfahren gemäss Anspruch 10, worin die Abgangsgruppe A Chlor, Brom, Iod oder Benzol- oder Toluolsulfonyloxy ist und M Wasserstoff oder ein Alkalimetall-, Erdalkalimetall- oder ein gegebenenfalls substituiertes Ammonium-Kation ist.

12. Verfahren gemäss Anspruch 10, dadurch gekennzeichnet, dass die Umsetzung in Gegenwart einer organischen oder anorganischen Base ausgeführt wird.

13. Verfahren gemäss Anspruch 10, dadurch gekennzeichnet, dass die Umsetzung in wässrig-alkalischem Medium ausgeführt wird und das entstandene Produkt durch Zusatz einer Mineralsäure ausgefällt wird.

14. Verfahren zur Korrosionsinhibierung wässriger Systeme, die mit Metall in Kontakt sind, durch Zusatz mindestens einer Verbindung der Formel I des Anspruchs 1.

15. Verfahren gemäss Anspruch 14, dadurch gekennzeichnet, dass man die Verbindung der Formel I in einer Menge von 0,1 ppm bis 5 Gew.%, bezogen auf das wässrige System, zusetzt.

16. Verfahren gemäss Anspruch 14, dadurch gekennzeichnet, dass das wässrige System ein Kühlwassersystem, eine Klimaanlage, eine Dampferzeugungsanlage, eine Seewasserverdampfungsanlage, ein Heizungs- oder Kühlwasserkreislauf oder eine wässrige Metallbearbeitungs-, Gefrierschutz-oder Hydraulik-Flüssigkeit ist.

17. Verfahren gemäss Anspruch 14, dadurch gekennzeichnet, dass ausser dem Korrosionsinhibitor der Formel I dem wässrigen System noch ein oder mehrere andere Korrosionsinhibitoren, Dispersionsmittel, Fällungsmittel, Sauerstoffabfänger, Komplexierungsmittel, Schaumdämpfer, Desinfektionsmittel, Hochdruckadditive oder sonstige bekannte Additive zugesetzt werden.

18. Verwendung von Verbindungen der Formel I des Anspruchs 1 als Korrosionsinhibitoren für wässrige Systeme.

19. Wässriges System, enthaltend als Korrosionsinhibitor mindestens eine Verbindung der Formel I des

Anspruchs 1 in einer Menge von 0,1 ppm bis 5 Gew.%, bezogen auf das wässrige System.

## Claims

**1.** A compound of the formula I

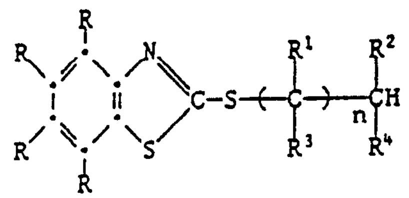

I

in which each R independently of the others is H, $C_1$-$C_{12}$alkyl, $C_1$-$C_{12}$alkoxy, $C_1$-$C_4$halogenalkyl, $C_1$-$C_{12}$alkylthio, $C_1$-$C_{12}$alkylsulfonyl, phenyl, -$C_7$-$C_{16}$alkylphenyl, $C_7$-$C_{16}$phenylalkyl, $C_5$-$C_8$cycloalkyl, halogen, -$NO_2$, -CN, -COOH, COO($C_1C_4$alkyl), -OH or a primary, secondary or tertiary amino or carbamoyl group having up to 20 C atoms, n is zero or 1, and, if n is 1, $R^1$, $R^2$, $R^3$ and $R^4$ independently of one another are H, $C_1$-$C_{18}$alkyl, $C_1$-$C_4$hydroxyalkyl, $C_2$-$C_{12}$carboxyalkyl, $C_1$-$C_4$halogenoalkyl, $C_2$-$C_{10}$alkoxyalkyl, -COON, phenyl or benzyl, or $R^1$ and $R^2$ or $R^1$ and $R^3$ together are linear or branched $C_3$-$C_8$alkylene which can be substituted by 1 or 2 carboxyl groups, or $R^1$ and $R^2$ together are a direct bond, and, if n is 0, $R^2$ and $R^4$ are carboxyl or are phenyl which is substituted by 1 or 2 carboxyl groups, subject to the proviso that the group

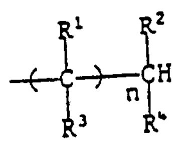

contains at least two and not more than four carboxyl groups, and a base addition salt of this compound.

**2.** A compound of the formula I, according to claim 1, in which one R is hydrogen, $C_1$-$C_4$alkyl, $C_1$-$C_4$alkoxy or amino and the other three Rs are hydrogen.

**3.** A compound according to claim 2, in which all four Rs are hydrogen.

**4.** A compound of the formula I, according to claim 1, in which two of the groups $R^1$, $R^2$, $R^3$ and $R^4$ are carboxyl or carboxyalkyl groups.

**5.** A compound of the formula I, according to claim 1, in which n is 1 and $R^1$, $R^2$, $R^3$ and $R^4$ independently of one another are H, $C_1$-$C_{12}$alkyl, $C_1$-$C_4$hydroxyalkyl, $C_2$-$C_6$carboxyalkyl, $C_2$-$C_{10}$alkoxyalkyl, carboxyl, phenyl or benzyl, or $R^1$ and $R^2$ together are trimethylene or tetramethylene.

**6.** A compound of the formula I, according to claim 5, in which n is 1 and $R^1$, $R^2$, $R^3$ and $R^4$ independently of one another are H, $C_1$-$C_4$alkyl, carboxyl or $C_2$-$C_6$carboxyalkyl.

**7.** A salt of a compound of the formula I, according to claim 1, which is a salt of an alkali metal, alkaline earth metal, metal of group IIB, IIIA or VIII of the periodic system, ammonia or an organic amine.

**8.** A salt according to claim 7, which is a sodium, potassium, ammonium, calcium, magnesium, zinc or aluminium salt or a salt of a primary, secondary or tertiary amine.

**9.** A compound according to claim 1 of the formulae

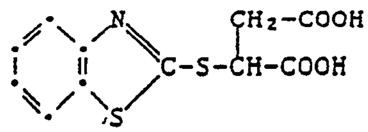,
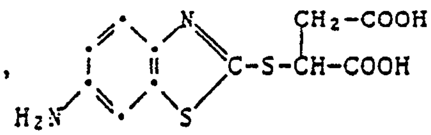

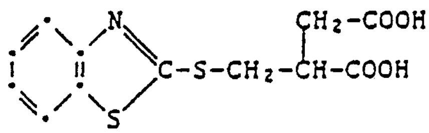 or

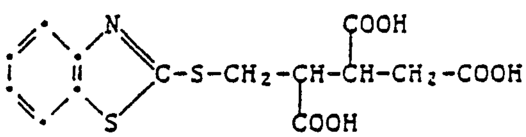 .

**10.** A process for the preparation of a compound of the formula I of claim 1, which comprises either reacting a compound of the formula II

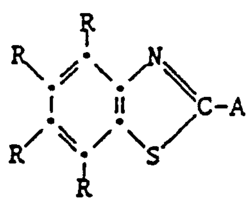

II

in which R is as defined in claim 1 and R is a leaving group, with a compound of the formula III

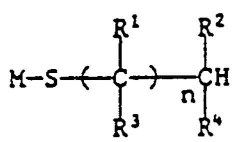 .

(III)

in which n, $R^1$, $R^2$, $R^3$ and $R^4$ are as defined in claim 1 and M is hydrogen or a cation, or reacting a compound of the formula IV

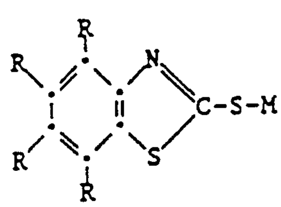

(IV)

with a compound of the formula V

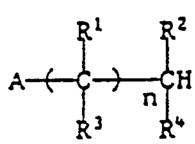

11. A process according to claim 10, in which the leaving group A is chlorine, bromine, iodine or benzenesulfonyloxy or toluenesulfonyloxy and M is hydrogen or an alkali metal or alkaline earth metal cation or a substituted or unsubstituted ammonium cation.

12. A process according to claim 10, wherein the reaction is carried out in the presence of an organic or inorganic base.

13. A process according to claim 10, wherein the reaction is carried out in an aqueous alkaline medium and the resulting product is precipitated by adding a mineral acid.

14. A process for inhibiting corrosion in aqueous systems in contact with metal, by adding at least one compound of the formula I of claim 1.

15. A process according to claim 14, wherein the compound of the formula I is added in an amount of 0.1 ppm to 5% by weight, based on the aqueous system.

16. A process according to claim 14, wherein the aqueous system is a cooling water system, an air-conditioning plant, a steam generating plant, a sea water evaporation plant, a heating or cooling water circulation system or an aqueous metal machining, anti-freeze or hydraulic fluid.

17. A process according to claim 14, wherein, in addition to the corrosion inhibitor of the formula I, one or more other corrosion inhibitors, dispersing agents, precipitants, oxygen acceptors, complexing agents, antifoaming agents, disinfectants, high-pressure additives or other known additives are also added to the aqueous system.

18. The use of a compound of the formula I of claim 1 as a corrosion inhibitor for aqueous systems.

19. An aqueous system containing, as a corrosion inhibitor, at least one compound of the formula I of claim 1 in an amount of 0.1 ppm to 5% by weight, based on the aqueous system.

## Revendications

1. Composés répondant à la formule I :

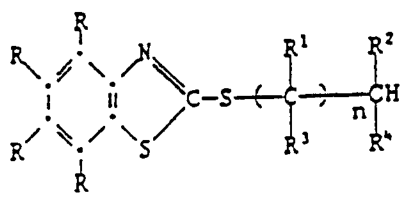

I

| | |
|---|---|
| les R | représentent chacun, indépendamment les uns des autres, l'hydrogène, un alkyle en $C_1$-$C_{12}$, un alcoxy en $C_1$-$C_{12}$, un halogéno-alkyle en $C_1$-$C_4$, un alkylthio en $C_1$-$C_{12}$, un alkylsulfonyle en $C_1$-$C_{12}$, un phényle, un alkylphényle en $C_7$-$C_{16}$, un phénylalkyle en $C_7$-$C_{16}$, un cycloalkyle en $C_5$-$C_8$, un halogène, $-NO_2$, -CN, -COOH, un alcoxycarbonyle à alkyle $C_1$-$C_4$, -OH ou un radical amino ou carbamoyle primaire, secondaire ou tertiaire contenant au plus 20 atomes de carbone, |
| n | est égal à 0 ou à 1 et - lorsque n est égal à 1 : |
| $R^1$, $R^2$, | $R^3$ et $R^4$ représentent chacun, indépendamment les uns des autres, H, un alkyle en $C_1$-$C_{18}$, un hydroxyalkyle en $C_1$-$C_4$, un carbo-alkyle en $C_1$-$C_{12}$, un halogéno-alkyle en $C_1$-$C_4$, un alcoxy-alkyle en $C_2$-$C_{10}$, -COOH, un phényle ou un benzyle, ou |
| $R^1$ et $R^2$ | , ou $R^1$ et $R^3$, forment ensemble un alkylène en $C_3$-$C_8$, ramifié ou non, qui peut porter 1 ou 2 radicaux carboxy, ou |
| $R^1$ et $R^2$ | forment ensemble une liaison directe, et - lorsque n est égal à 0 : |

$R^2$ et $R^4$ représentent chacun un carboxy ou un phényle qui porte 1 ou 2 radicaux carboxy, avec la condition que le radical :

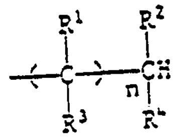

contienne au moins deux et au plus quatre radicaux carboxy,
ainsi que sels d'addition que ces composés forment avec des bases.

**2.** Composés de formule I selon la revendication 1 dans lesquels l'un des A représente l'hydrogène, un alkyle en $C_1$-$C_4$, un alcoxy en $C_1$-$C_4$ ou un amino et les trois autres symboles R représentent chacun l'hydrogène.

**3.** Composés selon la revendication 2 dans lesquels les quatre symboles R représentent chacun l'hydrogène.

**4.** Composés de formule I selon la revendication 1 dans lesquels deux des symboles $R^{\cdot}$, $R^2$, $R^2$ et $R^4$ représentent chacun un radical carboxy ou carboxy-alkyle.

**5.** Composés de formule I selon la revendication 1 dans lesquels n est égal à 1, $R^1$, $R^2$, $R^3$ et $R^4$ représentent chacun, indépendamment les uns des autres, l'hydrogène, un alkyle en $C_{\cdot}$-$C_{\cdot 2}$, un hydroxy-alkyle en $C_1$-$C_4$, un carboxy-alkyle en $C_2$-$C_6$, un alcoxy-alkyle en $C_2$-$C_{\cdot 0}$, un carboxy, un phényle ou un benzyle, ou $R^1$ et $R^2$ forment ensemble un radical triméthylène ou tétraméthylène.

**6.** Composés de formule I selon la revendication 5 dans lesquels n est égal à 1, et $R^{\cdot}$, $R^2$, $R^3$ et $R^4$ représentent chacun, indépendamment les uns des autres, l'hydrogène, un alkyle en $C_{\cdot}$-$C_4$, un carboxv ou un carboxy-alkyle en $C_2$-$C_6$.

**7.** Sels de composés de formule I selon la revendication 1, sels qui sont des sels de métaux alcalins, de métaux alcalinoterreux, de métaux des groupes IIB, IIIA ou VIII de la classification périodique, de l'ammoniac ou d'amines organiques.

**8.** Sels selon la revendication 7, qui sont des sels de sodium, de potassium, d'ammonium, de calcium, de magnésium, de zinc ou d'aluminium, ou des sels d'amines primaires, secondaires ou tertiaires.

**9.** Composé selon la revendication 1 qui répond à l'une des formules suivantes :

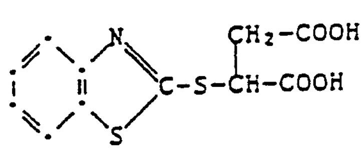 ,

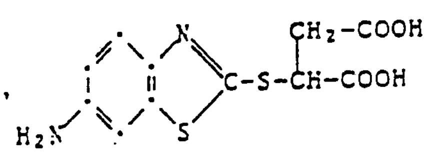 ,

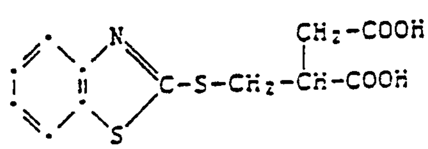

et

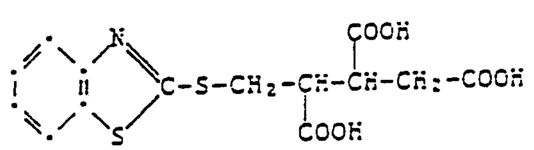 .

**10.** Procédé pour préparer des composés de formule I selon la revendication 1, procédé caractérisé en ce qu'on fait réagir soit un composé répondant à la formule II :

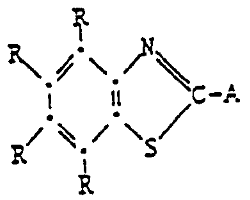

(II)

dans laquelle R a la signification indiquée à la revendication 1 et A représente un radical vinyle éliminable, avec un composé répondant à la formule III :

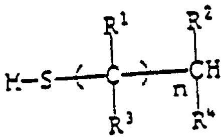

(III)

dans laquelle n, $R^1$, $R^2$, $R^3$ et $R^4$ ont les significations qui ont été données à la revendication 1 et M représente l'hydrogène ou un cation, soit un composé de formule IV :

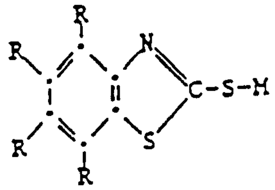

(IV)

avec un composé de formule V :

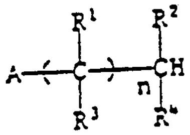

(V) .

**11.** Procédé selon la revendication 10 dans lequel le radical éliminable A est le chlore, le brome, l'iode ou un radical benzène-sulfonyloxy ou toluène-sulfonyloxy, et M représente l'hydrogène, un cation de métal alcalin, un cation de métal alcalino-terreux ou un cation d'ammonium éventuellement substitué.

**12.** Procédé selon la revendication 10 caractérisé en ce qu'on effectue la réaction en présence d'une base minérale ou organique.

**13.** Procédé selon la revendication 10 caractérisé en ce qu'on effectue la réaction dans un milieu aqueux alcalin et, par addition d'un acide minéral, on fait précipiter le produit qui s'est formé.

**14.** Procédé pour inhiber la corrosion de systèmes aqueux entrant en contact avec un métal, par addition d'au moins un composé de formule I selon la revendication 1.

**15.** Procédé selon la revendication 14 caractérisé en ce qu'on ajoute le composé de formule I en une quantité de 0,1 ppm à 5 % en poids par rapport au système aqueux.

**16.** Procédé selon la revendication 14 caractérisé en ce que le système aqueux est un système d'eau de refroidissement, une installation de climatisation, un générateur de vapeur, une installation d'évaporation d'eau de mer, un circuit d'eau de chauffage ou de refroidissement, un liquide aqueux pour l'usinage de métaux, un liquide aqueux anti-gel ou un liquide hydraulique aqueux.

**17.** Procédé selon la revendication 14 caractérisé en ce qu'on ajoute au système aqueux, en plus de l'inhibiteur de corrosion de formule I, un ou plusieurs autres inhibiteurs de corrosion, des dispersants, des agents de précipitation, des accepteurs d'oxygène, des complexants, des anti-mousses, des désinfectants, des additifs haute pression ou d'autres additifs connus.

**18.** Application de composés de formule I selon la revendication 1 comme inhibiteurs de corrosion pour des systèmes aqueux.

**19.** Système aqueux qui contient, comme inhibiteur de corrosion, au moins un composé de formule I selon la revendication 1 en une quantité de 0,1 ppm à 5 % en poids par rapport au système aqueux.